(19) Europäisches Patentamt

European Patent Office

Office européen des brevets

(11) EP 0 838 225 A2

(12) EUROPEAN PATENT APPLICATION

(43) Date of publication:
29.04.1998 Bulletin 1998/18

(51) Int. Cl.$^6$: A61K 47/48

(21) Application number: 97118411.4

(22) Date of filing: 23.10.1997

(84) Designated Contracting States:
AT BE CH DE DK ES FI FR GB GR IE IT LI LU MC NL PT SE

(30) Priority: 25.10.1996 JP 284470/96
14.11.1996 JP 303284/96
09.09.1997 JP 244093/97

(71) Applicants:
• Hiji, Yasutake
Yonago-shi, Tottori 683 (JP)
• Vitacain Pharmaceutical Co., Ltd.
Moriguchi-shi, Osaka 570 (JP)

(72) Inventor:
Hiji, Yasutake,
c/o Tottori University
Yonago-shi, Tottori 683 (JP)

(74) Representative:
von Kreisler, Alek, Dipl.-Chem. et al
Patentanwälte,
von Kreisler-Selting-Werner,
Bahnhofsvorplatz 1 (Deichmannhaus)
50667 Köln (DE)

(54) Aqueous local anesthetic solution

(57) An aqueous local anesthetic solution having a pH of 6.5-8.5, which contains a local anesthetic included in cyclodextrin or a derivative thereof in a concentration of the local anesthetic of not less than 0.02%, a method for improving solubility of local anesthetic in water at a pH of 6.5-8.5, comprising including the local anesthetic in cyclodextrin or a derivative thereof, a local anesthetic agent comprising an inclusion compound of local anesthetic in cyclodextrin or a derivative thereof, a method for decreasing neurotoxicity of local anesthetic agent, comprising including the local anesthetic in cyclodextrin or a derivative thereof, and an inclusion compound of local anesthetic in cyclodextrin or a derivative thereof. The local anesthetic agent and aqueous local anesthetic solution of the present invention have decreased neurotoxicity, so that the duration of anesthesia can be controlled clinically safely by reducing/increasing its concentration or dose. In particular, the aqueous local anesthetic solution can be used at a pH near that of a living body. Thus, it can be used even when living tissue has an acidic pH. Moreover, the pain caused by local acidosis at an injection site by a conventional local anesthetic dissolved in an acidic solution can be reduced.

EP 0 838 225 A2

**Description**

TECHNICAL FIELD OF THE INVENTION

The present invention relates to an aqueous local anesthetic solution containing a local anesthetic at a high concentration, a method for improving solubility of a local anesthetic in water, a local anesthetic agent with less neurotoxicity and a method for decreasing neurotoxicity of local anesthetic agents.

BACKGROUND OF THE INVENTION

Local anesthetics dissolve in acidic solvents, but are slightly soluble in neutral to alkaline solvents. For this reason, local anesthetics are generally formulated into acidic preparations. When such a local anesthetic agent is used for a non-alkaline tissue, such as tissues having an acidic pH due to inflammation, the local anesthetic agent cannot exert its effect to satisfaction. In addition, injection of a local anesthetic dissolved in an acidic solution into a living body induces sharp pain at the injection site due to local acidosis.

Thus, the development of a local anesthetic agent which dissolves in neutral to alkaline solvents and which can be used for a weak alkaline subject, such as a living body, has been desired.

An ideal local anesthetic is characterized by quick expression of anesthetic effect and sustained anesthesia, and various anesthetics have been developed In an attempt to afford a satisfactory local anesthetic. Ever since procaine having an anesthetic effect for a rather short time of 30 min to nearly one hour was synthesized, studies of local anesthetic have made rapid progress, and dibucaine, tetracaine and the like having strong anesthetic effect for a long time have been developed and used in a wide range of applications.

Anesthesia refers to a state of temporary abeyance of neurofunction and subsequent recovery to a normal state. It is known that, when a strong and long-acting anesthetic is used at a high concentration, it induces irreversible changes in nerve cells and neurofunction does not recover. In other words, a local anesthetic having a strong anesthetic effect is toxic to the tissue at the injection site and destroys nerve cells when used in high concentrations, so that dibucaine, tetracaine and the like require particular attention. Indeed, reports have been documented on irreversible block of vestibule function due to facial nerve block caused by local anesthetic and cerebrospinal function disorders resulting from spinal anesthesia.

Thus, the development has been awaited of a local anesthetic agent having less toxicity but quick in expression of anesthetic effect.

It is therefore an object of the present invention to provide a pharmaceutical agent containing a local anesthetic having strong anesthetic effect and less neurotoxicity, as well as a local anesthetic agent capable of quick expression of anesthetic effect.

It is another object of the present invention to provide a method of decreasing neurotoxicity caused by local anesthetic.

A yet another object of the present invention is to provide a pharmaceutical agent containing a local anesthetic which dissolves in neutral to alkaline solvents and which can be used for a weak alkaline subject such as a living body.

It is yet another object of the present invention to provide a method of improving solubility of local anesthetic in water.

It is yet another object of the present invention to provide a novel inclusion compound.

SUMMARY OF THE INVENTION

As a result of the study and investigation in an attempt to solve the above-mentioned problems, it has now been found that the use of a local anesthetic agent comprising a novel inclusion compound obtained by adding cyclodextrin or its derivative to a local anesthetic causes less neurotoxicity, while retaining superior anesthetic effect.

According to the present invention, it has been also found that inclusion of local anesthetic in cyclodextrin (hereinafter also referred to as CD) or its derivative results in markedly improved solubility of the local anesthetic, so that it can be dissolved in water in neutral to alkaline range, and an aqueous local anesthetic solution can be provided which can be used at a pH of about 7.4 which is the same as that in living body. What is more, superior anesthetic effect can be retained in said aqueous solution, even when neurotoxicity is decreased.

The present inventor has also found that the addition of salicylic acid or a salt thereof to an inclusion compound obtained by adding cyclodextrin or its derivative to a local anesthetic results in enhanced anesthetic effect of the local anesthetic, which in turn shortens the time necessary for expression of anesthesia and prolonged recovery time from anesthesia. That is, the present inventor has found that, by adding salicylic acid or a salt thereof to an inclusion compound of a local anesthetic in cyclodextrin or its derivative, a local anesthetic agent can be produced, which has an enhanced anesthetic effect and which is capable of expressing anesthetic effect quickly with a prolonged duration.

The present invention has been completed based on the above-mentioned new findings, and thus provides the following.

(1) Aqueous local anesthetic solutions having a pH of 6.5-8.5, which comprise a local anesthetic included in cyclodextrin or a derivative thereof in a concentration of the local anesthetic of not less than 0.02%, preferably not less than 0.05%.

(2) Aqueous local anesthetic solutions of the above (1), wherein the local anesthetic is lidocaine or a salt thereof, which is contained in a concentration of the lidocaine or a salt thereof of not less than 0.1%.

(3) Aqueous local anesthetic solutions of the above (1), wherein the local anesthetic is bupivacaine or a salt thereof, which is contained in a concentration of the bupivacaine or a salt thereof of not less than 0.1%.

(4) Aqueous local anesthetic solutions of the above (1), wherein the local anesthetic is dibucaine or a salt thereof, which is contained in a concentration of the dibucaine or a salt thereof of not less than 0.02%, preferably not less than 0.05%.

(5) Aqueous local anesthetic solutions of the above (1), wherein the local anesthetic is tetracaine or a salt thereof, which is contained in a concentration of the tetracaine or a salt thereof of not less than 0.02%, preferably not less than 0.05%.

(6) Aqueous local anesthetic solutions of any one of the above (1) to (5), further comprising salicylic acid or a salt thereof.

(7) External agents containing the aqueous local anesthetic solution of any one of the above (1) to (6).

(8) External agents of the above (7), which are either ointments, adhesive plasters or a suppositories.

(9) Methods for improving solubility of a local anesthetic in water at pH 6.5-8.5, comprising including the local anesthetic in cyclodextrin or a derivative thereof.

(10) Methods for improving solubility of the above (9), wherein the local anesthetic is at least one member selected from the group consisting of lidocaine, bupivacaine, dibucaine, tetracaine and salts thereof.

(11) Local anesthetic agents comprising an inclusion compound of a local anesthetic in α-cyclodextrin or a derivative thereof.

(12) Local anesthetic agents of the above (11) having a decreased neurotoxicity.

(13) Local anesthetic agents of the above (11), wherein the local anesthetic is at least one member selected from the group consisting of dibucaine, tetracaine and salts thereof.

(14) Local anesthetic agents of any one of the above (11) to (13), further comprising salicylic acid or a salt thereof.

(15) Methods of decreasing neurotoxicity of a local anesthetic agent, comprising including the local anesthetic in α-cyclodextrin or a derivative thereof.

(16) Inclusion compounds of local anesthetic in α-cyclodextrin or a derivative thereof.

(17) Inclusion compounds of the above (16), wherein the local anesthetic is at least one member selected from the group consisting of dibucaine, tetracaine and salts thereof.

(18) Local anesthetic agents comprising an inclusion compound of tetracaine or a salt thereof in β-cyclodextrin or a derivative thereof.

(19) Local anesthetic agents of the above (18), having decreased neurotoxicity.

(20) Local anesthetic agents of the above (18) or (19), further comprising salicylic acid or a salt thereof.

(21) Methods of decreasing neurotoxicity of tetracaine or a salt thereof, comprising including tetracaine or a salt thereof in β-cyclodextrin or a derivative thereof.

(22) Inclusion compounds of tetracaine or a salt thereof in β-cyclodextrin or a derivative thereof.

(23) Local anesthetic agents comprising an inclusion compound of lidocaine or a salt thereof in β-cyclodextrin, γ-cyclodextrin or a derivative thereof.

(24) Local anesthetic agents of the above (23), having decreased neurotoxicity.

(25) Local anesthetic agents of the above (23) or (24), further comprising salicylic acid or a salt thereof.

(26) Methods of decreasing neurotoxicity of lidocaine or a salt thereof, comprising including lidocaine or a salt thereof in β-cyclodextrin, γ-cyclodextrin or a derivative thereof.

(27) Inclusion compounds of lidocaine or a salt thereof in β-cyclodextrin, γ-cyclodextrin or a derivative thereof.

BRIEF DESCRIPTION OF THE DRAWINGS

Fig. 1 shows absorption spectra of solutions prepared by adding α-cyclodextrin to dibucaine solutions.
Fig. 2 shows results of difference spectrum analysis of solutions prepared by adding α-cyclodextrin to dibucaine solutions.
Fig. 3 shows absorption spectra of solutions prepared by adding β-cyclodextrin to dibucaine solutions.
Fig. 4 shows results of difference spectrum analysis of solutions prepared by adding β-cyclodextrin to dibucaine solutions.

Fig. 5 shows results of difference spectrum analysis of solutions prepared by adding γ-cyclodextrin to dibucaine solutions.

Fig. 6 shows absorption spectra of solutions prepared by adding α-cyclodextrin to tetracaine solutions.

Fig. 7 shows results of difference spectrum analysis of solutions prepared by adding α-cyclodextrin to tetracaine solutions.

Fig. 8 shows absorption spectra of solutions prepared by adding β-cyclodextrin to tetracaine solutions.

Fig. 9 shows results of difference spectrum analysis of solutions prepared by adding β-cyclodextrin to tetracaine solutions.

Fig. 10 shows absorbance at wavelength 313 nm of difference spectrum of inclusion compounds obtained by adding various concentrations of cyclodextrins (α-cyclodextrin, β-cyclodextrin, γ-cyclodextrin) to 0.25 mM dibucaine.

Fig. 11 shows the relationship between lidocaine concentration and action potential disappearance time (block time) of aodominal giant nerve of crayfish (*Procambarus clarkii*) when lidocaine solutions having different pHs and a lidocaine solution containing 1% sodium salicylate were used.

Fig. 12 shows the relationship between dibucaine concentration and action potential disappearance time of abdominal giant nerve of crayfish when dibucaine was used alone or when sodium salicylate and/or α-cyclodextrin were/was added to dibucaine.

Fig. 13 shows the relationship between dibucaine concentration and action potential recovery time of abdominal giant nerve of crayfish when dibucaine was used alone or when sodium salicylate and/or α-cyclodextrin were/was added to dibucaine.

Fig. 14 shows action potential disappearance time, recovery time and maximum recovery percentage of abdominal giant nerve of crayfish when a lidocaine solution was used alone and when a solution of an inclusion compound of lidocaine in hydroxypropyl-β-cyclodextrin was used.

Fig. 15 shows action potential disappearance time, recovery time and maximum recovery percentage of abdominal giant nerve of crayfish when a lidocaine solution containing sodium salicylate was used and when a solution of an inclusion compound of lidocaine in hydroxypropyl-β-cyclodextrin and containing sodium salicylate was used.

Fig. 16 shows anesthesia effective concentration, recovery concentration and safety ratio when a lidocaine solution was used alone, when a solution of an inclusion compound of lidocaine in hydroxypropyl-β-cyclodextrin was used and when a solution of an inclusion compound of lidocaine in hydroxypropyl-γ-cyclodextrin was used.

Fig. 17 shows anesthesia effective concentration, recovery concentration and safety ratio when a lidocaine solution containing sodium salicylate was used, when a solution of an inclusion compound of lidocaine in hydroxypropyl-β-cyclodextrin and containing sodium salicylate was used and when a solution of an inclusion compound of lidocaine in hydroxypropyl-γ-cyclodextrin and containing sodium salicylate was used.

DETAILED DESCRIPTION OF THE INVENTION

While the local anesthetic to be used in the present invention is not particularly limited, it is preferably lidocaine, bupivacaine, dibucaine, tetracaine, mepivacaine and the like.

Salts of these local anesthetics may be also used, which are preferably hydrochlorides.

The cyclodextrin to be used in the present invention is exemplified by α-cyclodextrin, β-cyclodextrin, γ-cyclodextrin and the like.

Derivatives of these cyclodextrins may be also used. The derivative of cyclodextrin to be used in the present invention is exemplified by compounds having a ring structure consisting of 6 glucose units and a substituent having a hydroxyl group, such as glucosyl-α-cyclodextrin, diglucosyl-α-cyclodextrin, maltosyl-α-cyclodextrin, dimaltosyl-α-cyclodextrin, hydroxypropyl-α-cyclodextrin and the like; compounds having a ring structure consisting of 7 glucose units and a substituent having a hydroxyl group, such as glucosyl-β-cyclodextrin, diglucosyl-β-cyclodextrin, maltosyl-β-cyclodextrin, dimaltosyl-β-cyclodextrin, hydroxypropyl-β-cyclodextrin and the like; and compounds having a ring structure consisting of 8 glucose units and a substituent having a hydroxyl group, such as hydroxypropyl-γ-cyclodextrin and the like.

The aqueous local anesthetic solution and local anesthetic agent of the present invention can be prepared by dissolving an inclusion compound of a local anesthetic in cyclodextrin or a derivative thereof, in water having a pH of 6.0-8.6, preferably 6.5-8.5, more preferably about 7.4.

The solvent to be used for producing the local anesthetic agent of the present invention may be other than water.

The inclusion compound of local anesthetic in cyclodextrin or a derivative thereof in the aqueous local anesthetic solution and local anesthetic agent of the present invention can be produced by a method known *per se*. To be specific, a local anesthetic and cyclodextrin or a derivative thereof are brought into contact in the presence of water.

The inclusion compound to be used for the preparation of the local anesthetic agent of the present invention may be prepared by bringing the above-mentioned two into contact in a solvent other than water.

The temperature during the contact is generally 60-70°C, preferably 65°C. The amount to be added of cyclodextrin or a derivative thereof is generally not less than 0.2 mole, preferably 0.5 mole to 3 moles, per mole of a local anesthetic.

Inclusion of a local anesthetic in cyclodextrin or a derivative thereof leads to noticeable improvement in the solubility of local anesthetic, thereby enabling dissolution of said inclusion compound in water at pH 6.0-8.6, preferably 6.5-8.5.

Alternatively, cyclodextrin or a derivative thereof may be added directly to a solution containing a local anesthetic and having a pH of 6.0-8.6, preferably 6.5-8.5, and more preferably about pH 7.4, and ultrasonicated at 60-70°C, preferably 65°C for dissolution, to give the aqueous local anesthetic solution and local anesthetic agent of the present invention. In this case, cyclodextrin or a derivative thereof is generally added in a proportion of not less than 0.2 mole, preferably 0.5 mole to 3 moles, per mole of a local anesthetic.

The aqueous local anesthetic solution of the present invention contains a local anesthetic included in cyclodextrin or a derivative thereof in a concentration of the local anesthetic alone of not less than 0.02%, preferably not less than 0.05%.

The aqueous local anesthetic solution of the present invention thus obtained has a pH of 6.0-8.6, preferably 6.5-8.5, and can be used in the same weak alkaline range as the living body. Consequently, it can exert local anesthetic effect sufficiently even when biological tissue is acidic due to inflammation and the like. Inasmuch as the aqueous local anesthetic solution of the present invention has a pH of 6.0-8.6, preferably 6.5-8.5, a strong pain caused by local acidosis as observed upon injection of conventional local anesthetic dissolved in an acidic solution can be alleviated and thus, is clinically useful.

The local anesthetic agent of the present invention thus obtained has markedly reduced neurotoxicity as compared to conventional local anesthetics, and dissolves well in water having an about neutral pH (e.g., pH 6.5-7.5) to give superior pharmaceutical preparations.

The aqueous local anesthetic solution and inclusion compound of local anesthetic in cyclodextrin or a derivative thereof, obtained in the above, may contain salicylic acid or a salt thereof. The concentration of salicylic acid or a salt thereof is generally 0.01-4 wt%, preferably 0.05-1 wt%, of said inclusion compound, and it is not less than 0.01 wt%, preferably 0.05-5 wt%, of said aqueous local anesthetic solution. Addition of salicylic acid or a salt thereof results in the provision of aqueous local anesthetic solution and local anesthetic agent having enhanced anesthetic effect and capable of expressing anesthetic effect quickly for a prolonged duration of time.

The aqueous local anesthetic solution and local anesthetic agent may contain additives generally acceptable for preparations. Examples of the additive include carriers, stabilizers (e.g., creatinine), solubilizers (e.g., glycerol and glucose), suspending agents (e.g., CMC), buffers (e.g., citric acid and sodium hydrogencarbonate), emulsifiers (e.g., fatty acid monoglyceride, sorbitan esters of fatty acid and polyoxyethylene laurylether), preservatives (e.g., methyl and propyl parahydroxybenzoates), antioxidants (e.g., BHA and BHT) and the like.

After admixing various ingredients, the resulting mixture is prepared by a known method into dosage forms suitable for intended administration, such as injections, liquids, sprays, jelly and the like, in a solution, suspension or emulsion state, or powdery preparations which can be dissolved, suspended or emulsified when in use to give an injection or liquid.

It is also possible to prepare a powdery preparation which can be dissolved when in use to give an injection or liquid, and prepare the aqueous local anesthetic solution of the present invention upon dissolution of the powdery preparation.

Such powdery preparation can further contain suitable excipients.

The aqueous local anesthetic solution of the present invention may be prepared into external agents such as ointments, plasters, suppositories and the like.

Examples of ointment include, gel, oily ointment, emulsion ointment, aqueous ointment and the like, which can be prepared by a conventional method by admixing the aqueous local anesthetic solution with a base material such as fat, fatty oil, lanolin, petrolatum, paraffin, wax, resin, glycols, higher alcohols, glycerol, alcohols and water.

Suppositories can be prepared by a conventional method by admixing the aqueous local anesthetic solution with a base material such as cacao butter, lanolin oil, macrogol, sodium carboxymethylcellulose, methylcellulose, glycerol and the like.

Adhesive plasters can be prepared by a conventional method by adding, for example, glycerol, polyethylene glycol, carbonyl vinyl polymer, gelatin, zinc oxide, kaolin, sodium polyacrylate and the like, to the aqueous local anesthetic solution.

External agents may contain the aforementioned stabilizers and the like.

The external agents contain a local anesthetic included in cyclodextrin or a derivative thereof, in a concentration of the local anesthetic of not less than 0.1%, preferably 0.2-10%.

The aqueous local anesthetic solution of the present invention may additionally contain other efficacious ingredients such as antihistamines, nonsteroidal antiinflammatory drugs, adrenocorticosteroids, aromatics, blood circulation improving agents, antipruritic and the like.

While the dose and concentration in use of the aqueous local anesthetic solution and local anesthetic agent vary depending on age and symptom of patients, administration site, drugs to be used and the like, the dose is, as defined in the amount of the local anesthetic contained, 0.2-300 mg, preferably 0.5-200 mg, and the concentration in use is 0.01-5%, preferably 0.03-3% of the local anesthetic, when an aqueous local anesthetic solution containing a local anesthetic included in cyclodextrin or a derivative thereof is administered to an adult.

In the case of an aqueous local anesthetic solution and local anesthetic agent containing an inclusion compound of dibucaine in α-cyclodextrin, for example, the concentration in use for infiltration anesthesia is 0.01-0.3%, preferably 0.03-0.1%, of dibucaine. When they are used for spinal anesthesia, the dose is 1-15 mg, preferably 2-10 mg, of dibucaine and concentration in use is 0.05-0.5%, preferably 0.1-0.3%, of dibucaine for an adult.

In the case of a local anesthetic agent containing an inclusion compound of tetracaine in β-cyclodextrin, for example, the concentration in use for infiltration anesthesia is 0.02-0.3%, preferably 0.05-0.2%, of tetracaine. When it is used for spinal anesthesia, the dose is 2-20 mg, preferably 3-15 mg, of tetracaine and concentration in use is 0.05-1.0%, preferably 0.1-0.5%, of tetracaine for an adult.

When an ointment containing an aqueous local anesthetic solution containing an inclusion compound of lidocaine hydrochloride in cyclodextrin or a derivative thereof is used in a concentration of about 1-5% of lidocaine, the ointment is preferably administered 1-3 times a day according to the symptom.

The present invention is described in more detail by way of Examples, which should not be construed as limiting the invention.

Example 1

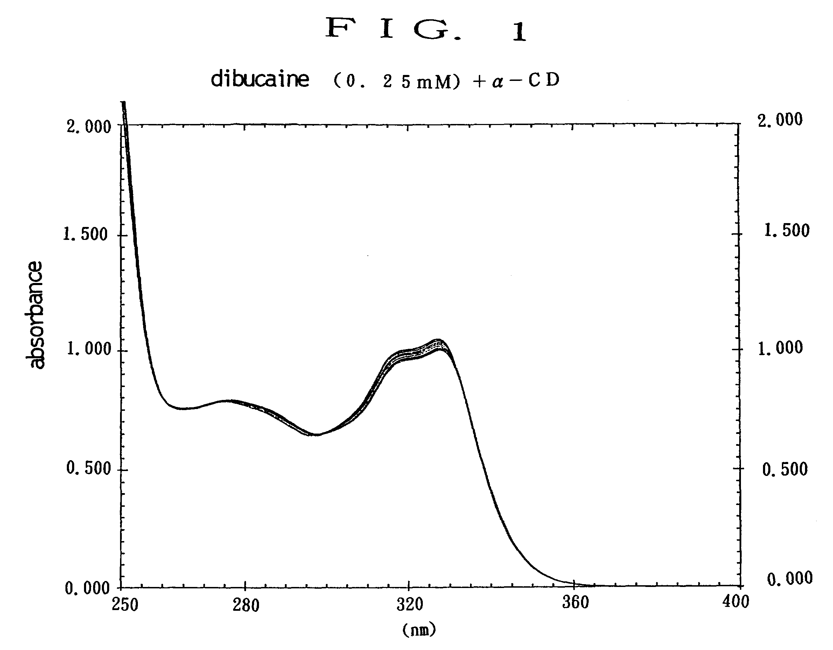

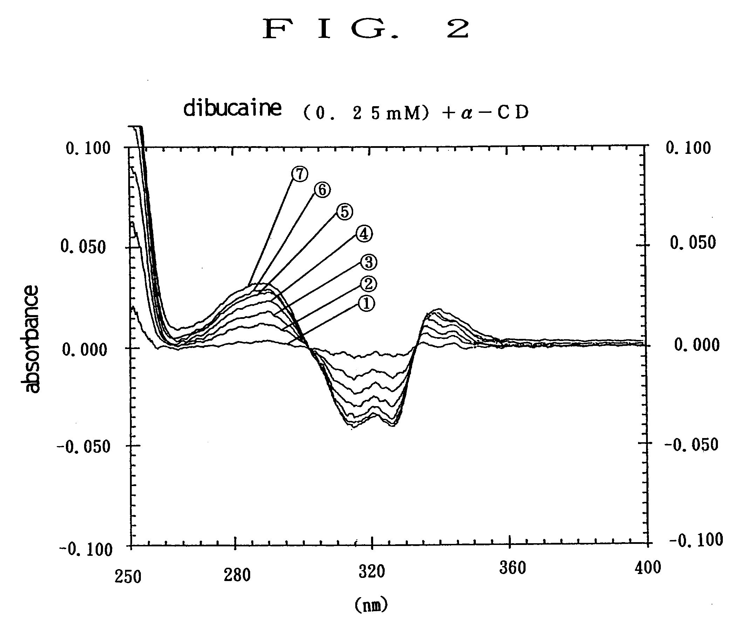

Various concentrations (0, 0.425, 1.05, 2.06, 3.96, 7.35, 10.29, 15.13 mM) of α-cyclodextrin (hereinafter also referred to as α-CD) were completely dissolved in 0.25 mM dibucaine solution (pH 7.4), and ultrasonicated at 60-65°C for 2-3 min. After confirmation of the solution becoming completely clear, the solution was placed in 2.4 ml determination cells, which were subjected to determination of difference spectrum using a spectrophotometer and analysis. The absorption spectra obtained by the determination are shown in Fig. 1, and changes in absorption spectra were enlarged by difference spectrum analysis, the results of which are shown in Fig. 2. From the obtained results noting changes in absorption spectra, it was confirmed that inclusion compounds of dibucaine in various concentrations of α-cyclodextrin were obtained.

In Fig. 2, ①-⑦ show analysis results of the following inclusion compounds.

① : 0.25 mM dibucaine+0.425 mM α-cyclodextrin
② : 0.25 mM dibucaine+1.05 mM α-cyclodextrin
③ : 0.25 mM dibucaine+2.06 mM α-cyclodextrin
④ : 0.25 mM dibucaine+3.96 mM α-cyclodextrin
⑤ : 0.25 mM dibucaine+7.35 mM α-cyclodextrin
⑥ : 0.25 mM dibucaine+10.29 mM α-cyclodextrin
⑦ : 0.25 mM dibucaine+15.13 mM α-cyclodextrin

Example 2

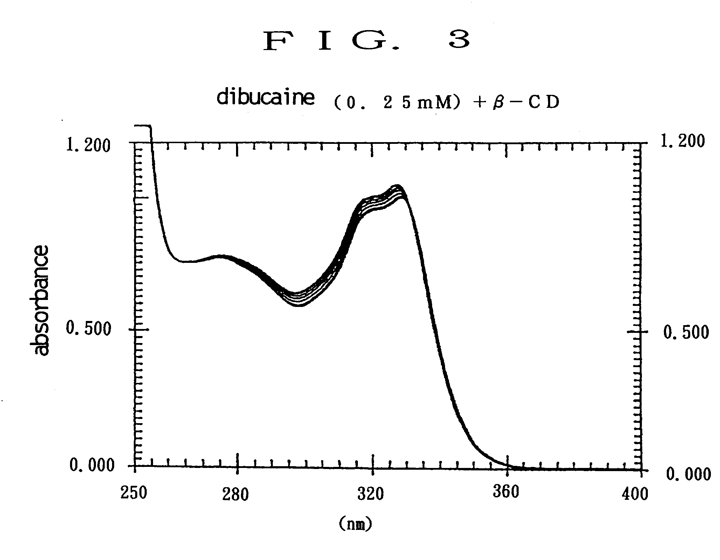

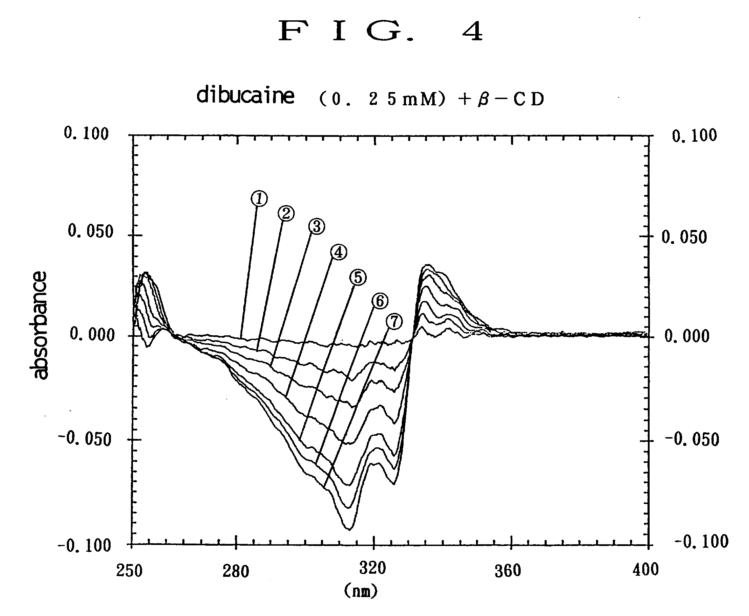

Various concentrations (0, 0.11, 0.54, 1.06, 2.04, 3.78, 5.29, 7.78 mM) of β-cyclodextrin (hereinafter also referred to as β-CD) were dissolved in 0.25 mM dibucaine solution (pH 7.4), and treated in the same manner as in Examples 1 and 2. The obtained solutions were analyzed by difference spectrum method using a spectrophotometer. The absorption spectra obtained by the determination are shown in Fig. 3. Changes in absorption spectra were enlarged by difference spectrum analysis, the results of which are shown in Fig. 4. From the obtained results noting changes in absorption spectra, it was confirmed that inclusion compounds of dibucaine in various concentrations of β-cyclodextrin were obtained.

In Fig. 4, ①-⑦ show analysis results of the following inclusion compounds.

① : 0.25 mM dibucaine+0.11 mM β-cyclodextrin
② : 0.25 mM dibucaine+0.54 mM β-cyclodextrin
③ : 0.25 mM dibucaine+1.06 mM β-cyclodextrin
④ : 0.25 mM dibucaine+2.04 mM β-cyclodextrin
⑤ : 0.25 mM dibucaine+3.78 mM β-cyclodextrin
⑥ : 0.25 mM dibucaine+5.29 mM β-cyclodextrin
⑦ : 0.25 mM dibucaine+7.78 mM β-cyclodextrin

Example 3

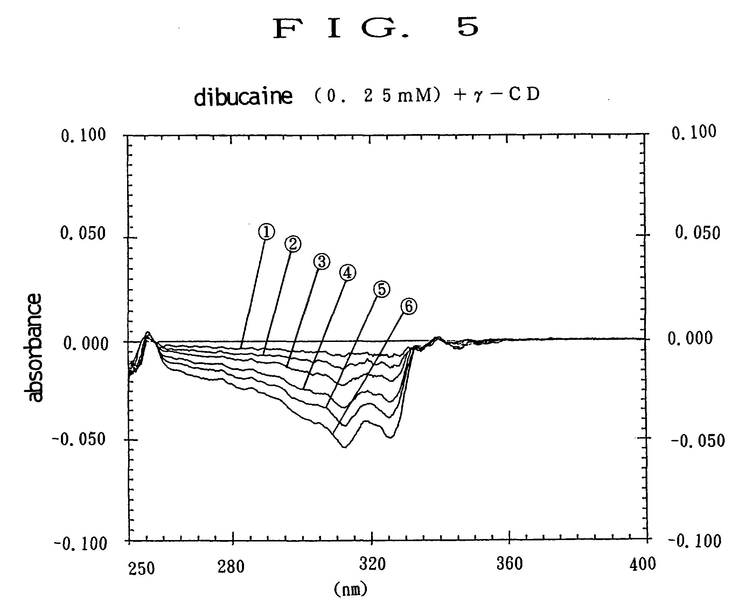

Various concentrations (0, 1.10, 2.16, 4.16, 7.72, 10.80, 15.89 mM) of γ-cyclodextrin (hereinafter also referred to as γ-CD) were dissolved in 0.25 mM dibucaine solution (pH 7.4), and treated in the same manner as in Examples 1. The obtained solutions were analyzed by difference spectrum method using a spectrophotometer. The results are shown in Fig. 5. From the obtained results noting changes in absorption spectra, it was confirmed that inclusion compounds of dibucaine in various concentrations of γ-cyclodextrin were obtained.

In Fig. 5, ①-⑥ show analysis results of the following inclusion compounds.

    ① : 0.25 mM dibucaine+1.10 mM γ-cyclodextrin
    ② : 0.25 mM dibucaine+2.16 mM γ-cyclodextrin
    ③ : 0.25 mM dibucaine+4.16 mM γ-cyclodextrin
    ④ : 0.25 mM dibucaine+7.72 mM γ-cyclodextrin
    ⑤ : 0.25 mM dibucaine+10.80 mM γ-cyclodextrin
    ⑥ : 0.25 mM dibucaine+15.89 mM γ-cyclodextrin

Example 4

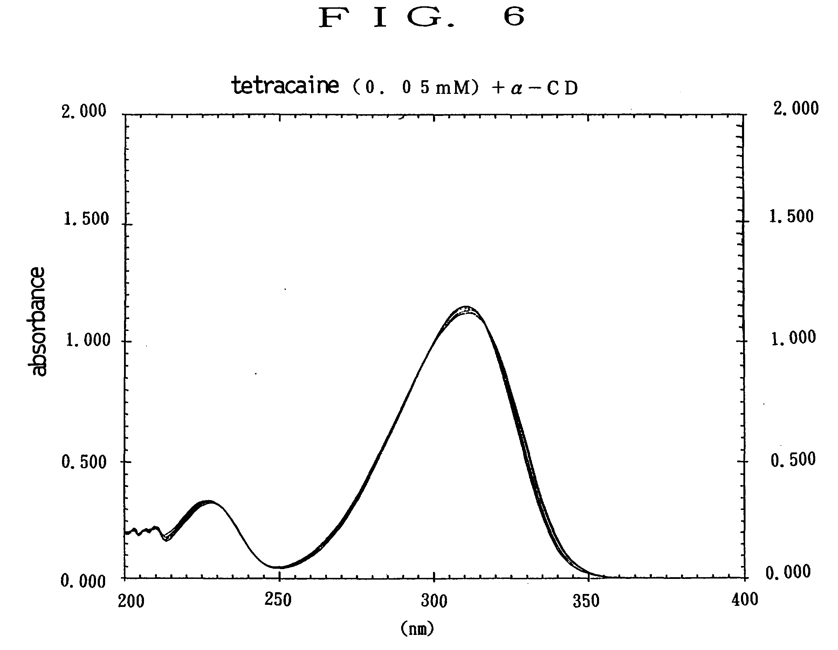

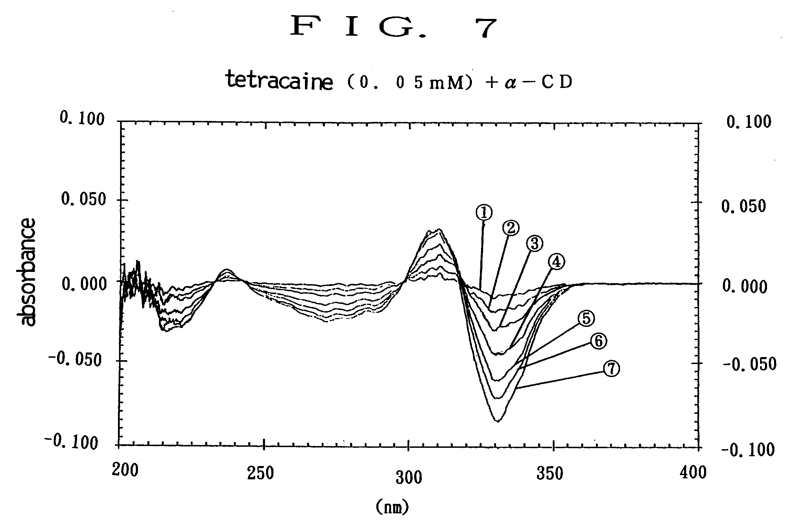

Various concentrations (0, 0.425, 1.05, 2.06, 3.96, 7.35, 10.29, 15.13 mM) of α-cyclodextrin were dissolved in 0.05 mM tetracaine solution (pH 7.4), and treated in the same manner as in Example 1. The obtained solutions were analyzed by difference spectrum method using a spectrophotometer. The absorption spectra obtained by the determination are shown in Fig. 6. Changes in absorption spectra were enlarged by difference spectrum analysis, the results of which are shown in Fig. 7. From the obtained results noting changes in absorption spectra, it was confirmed that inclusion compounds of tetracaine in various concentrations of α-cyclodextrin were obtained.

In Fig. 7, ①-⑦ show analysis results of the following inclusion compounds.

    ① : 0.05 mM tetracaine+0.425 mM α-cyclodextrin
    ② : 0.05 mM tetracaine+1.05 mM α-cyclodextrin
    ③ : 0.05 mM tetracaine+2.06 mM α-cyclodextrin
    ④ : 0.05 mM tetracaine+3.96 mM α-cyclodextrin
    ⑤ : 0.05 mM tetracaine+7.35 mM α-cyclodextrin
    ⑥ : 0.05 mM tetracaine+10.29 mM α-cyclodextrin
    ⑦ : 0.05 mM tetracaine+15.13 mM α-cyclodextrin

Example 5

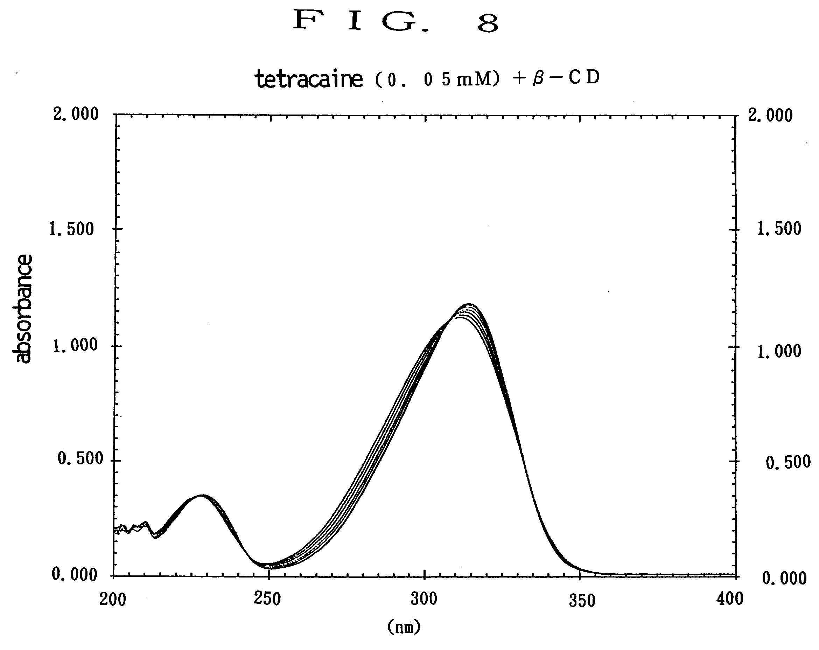

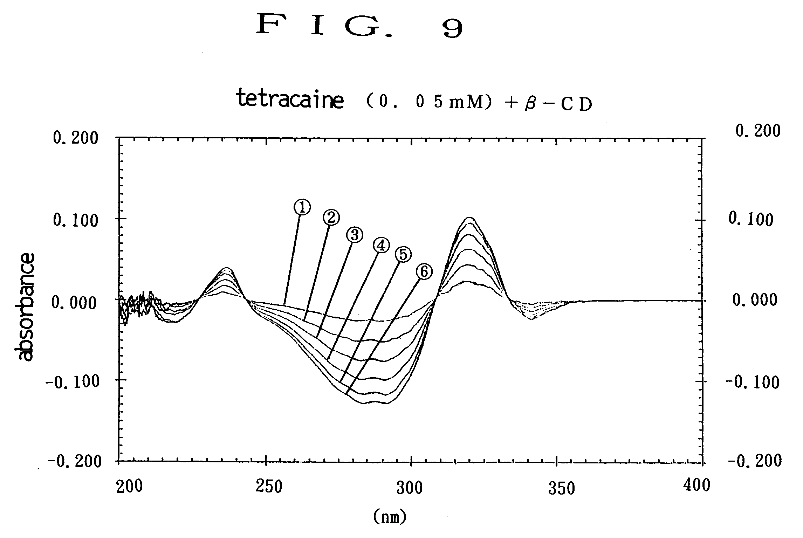

Various concentrations (0, 0.22, 0.54, 1.06, 2.04, 3.78, 6.61 mM) of β-cyclodextrin were dissolved in 0.05 mM tetracaine solution (pH 7.4), and treated in the same manner as in Example 1. The obtained solutions were analyzed by difference spectrum method using a spectrophotometer. The absorption spectra obtained by the determination are shown in Fig. 8. Changes in absorption spectra were enlarged by difference spectrum analysis, the results of which are shown in Fig. 9. From the obtained results noting changes in absorption spectra, it was confirmed that inclusion compounds of tetracaine in various concentrations of β-cyclodextrin were obtained.

In Fig. 9, ①-⑥ show analysis results of the following inclusion compounds.

    ① : 0.05 mM tetracaine+0.22 mM β-cyclodextrin
    ② : 0.05 mM tetracaine+0.54 mM β-cyclodextrin
    ③ : 0.05 mM tetracaine+1.06 mM β-cyclodextrin
    ④ : 0.05 mM tetracaine+2.04 mM β-cyclodextrin
    ⑤ : 0.05 mM tetracaine+3.78 mM β-cyclodextrin
    ⑥ : 0.05 mM tetracaine+6.61 mM β-cyclodextrin

While cyclodextrin has a ring structure consisting of several glucose molecules, various local anesthetics are included in cavities therein to form new complexes, as is evident from the changes in absorption spectra in Examples 1-5.

Example 6

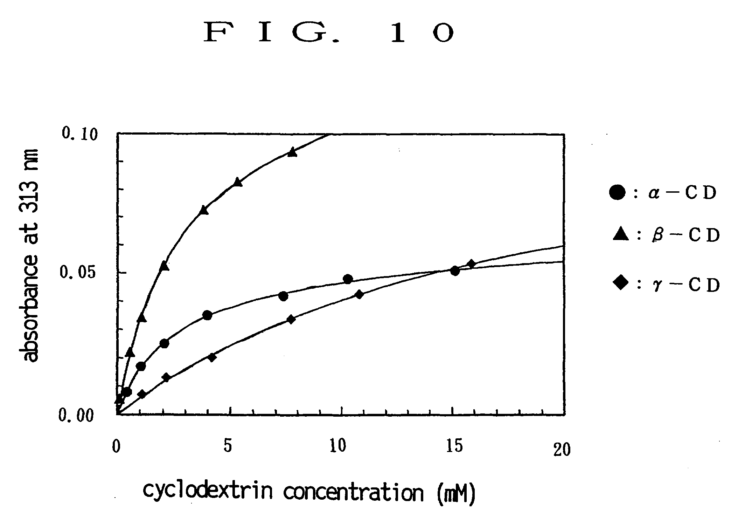

The chemical properties of the above-mentioned inclusion complexes were examined by reading, from the results of Examples 1-3, the level of absorbance of difference spectra at wavelength 313 nm obtained when various concentrations of cyclodextrin (α-CD, β-CD, γ-CD) in Examples 1-3 were added to 0.25 mM dibucaine solutions, and shown in the graph of Fig. 10.

It is clear that dibucaine formed new complexes by being included in cyclodextrin, and that the mode of formation varied according to the kind of $\alpha$-CD, $\beta$-CD and $\gamma$-CD.

Formulation Example 1

| 0.1% dibucaine hydrochloride injection (100 ml) | |
|---|---|
| dibucaine hydrochloride | 100 mg |
| $\alpha$-cyclodextrin | 500 mg |
| 1N aqueous sodium hydroxide solution | q.s. |
| distilled water | q.s. |
| The pH is adjusted to 7.5 with 1N aqueous sodium hydroxide solution. | |

Formulation Example 2

| 1% lidocaine hydrochloride injection (100 ml) | |
|---|---|
| lidocaine hydrochloride | 1 g |
| hydroxypropyl-$\beta$-cyclodextrin | 8 g |
| 1N aqueous sodium hydroxide solution | q.s. |
| distilled water | q.s. |
| The pH is adjusted to 7.4 with 1N aqueous sodium hydroxide solution. | |

Formulation Example 3

| ointment (total amount 100 g) | |
|---|---|
| crotamiton | 5 g |
| diphenhydramine | 1 g |
| l-menthol | 3 g |
| dl-camphor | 2 g |
| lidocaine | 2 g |
| hydroxypropyl-$\beta$-cyclodextrin | 5 g |
| sodium salicylate | 1 g |
| carboxyvinyl polymer | 0.4 g |
| macrogol 400 | 35 g |
| isopropanol | 35 g |
| hydrochloric acid | q.s. |
| sodium hydroxide | q.s. |
| purified water | q.s. |

Formulation Example 4

| ointment (total amount 100 g) | |
|---|---|
| dibucaine hydrochloride | 1 g |
| chlorpheniramine | 0.5 g |
| l-menthol | 4 g |
| dl-camphor | 2 g |
| Hibis Waco 104 | 1.5 g |
| diisopropyl adipate | 1.5 g |
| polyethylene glycol | 3.2 g |
| ethanol | 20 g |
| propylene glycol | 35 g |
| $\gamma$-cyclodextrin | 3 g |
| sodium hydroxide | q.s. |
| purified water | q.s. |

Formulation Example 5

| plaster (total amount 100 g) | |
|---|---|
| lidocaine | 10 g |
| sodium salicylate | 10 g |
| hydroxypropyl-$\beta$-cyclodextrin | 30 g |
| gelatin | 3 g |
| zinc oxide | 7 g |
| carboxyvinyl polymer | 2 g |
| polyethylene glycol 400 | 5 g |
| glycerol | 20 g |
| hydrochloric acid | q.s. |
| sodium hydroxide | q.s. |
| purified water | q.s. |

Formulation Example 6

| suppository (suppository 1.5 g) | |
|---|---|
| hydrocortisone acetate | 5 mg |
| tocopherol acetate | 40 mg |
| dibucaine hydrochloride | 4 mg |
| allantoin | 15 mg |
| chlorhexidine hydrochloride | 4 mg |
| $\alpha$-cyclodextrin | 20 mg |
| base material for suppository | q.s. |

Formulation Example 7

| injection 100 ml | |
|---|---|
| dibucaine hydrochloride | 300 mg |
| $\alpha$-cyclodextrin | 1000 mg |
| sodium chloride (isotonizing agent) | q.s. |
| sodium hydroxide (pH adjusting agent) | q.s. |
| water for injection | q.s. |

Formulation Example 8

| injection 100 ml | |
|---|---|
| dibucaine hydrochloride | 100 mg |
| sodium salicylate | 300 mg |
| $\alpha$-cyclodextrin | 500 mg |
| sodium chloride | q.s. |
| sodium hydroxide (pH adjusting agent) | q.s. |
| hydrochloric acid (pH adjusting agent) | q.s. |
| water for injection | q.s. |

Formulation Example 9

| injection 100 ml | |
|---|---|
| tetracaine hydrochloride | 500 mg |
| $\beta$-cyclodextrin | 2000 mg |
| sodium chloride (isotonizing agent) | q.s. |
| sodium hydroxide (pH adjusting agent) | q.s. |
| water for injection | q.s. |

Formulation Example 10

| injection 100 ml | |
|---|---|
| tetracaine hydrochloride | 100 mg |
| sodium salicylate | 500 mg |
| β-cyclodextrin | 500 mg |
| sodium chloride (isotonizing agent) | q.s. |
| sodium hydroxide (pH adjusting agent) | q.s. |
| water for injection | q.s. |

Experimental Example 1

To a 0.2% dibucaine solution (pH 7.25) were added α-cyclodextrin, β-cyclodextrin and γ-cyclodextrin, respectively having an equimolar concentration, and inclusion compound solutions were prepared in the same manner as in Example 1. The obtained inclusion compound solutions of dibucaine in α-cyclodextrin, β-cyclodextrin and γ-cyclodextrin, and 0.2% dibucaine single solution were examined for absorbance at wavelength 620 nm [OD (620 nm)] using UV-visible recording spectrophotometer (UV-2100, Shimadzu Corporation). The turbidity and clearness of each inclusion compound solution were calculated from the absorbances thus obtained. The results are shown in Table 1.

Then, to 0.5% tetracaine solution (pH 7.75), 0.5% bupivacaine solution (pH 7.25) and 2.0% lidocaine solution (pH 7.75) were respectively added α-cyclodextrin, β-cyclodextrin, hydroxypropyl-β-cyclodextrin (hereinafter also referred to as HP-β-CD) and hydroxypropyl-γ-cyclodextrin (hereinafter also referred to as HP-γ-CD), all of which having an equimolar concentration, and inclusion compound solutions were prepared in the same manner as in Example 1. In the same manner as above, OD (620 nm) was determined with respect to the obtained inclusion compound solution of each local anesthetic in cyclodextrin or a derivative thereof, and solutions of each local anesthetic. The turbidity and clearness of each inclusion compound solution were calculated from the absorbances thus obtained. The results are shown in Table 1.

Table 1

| local anesthetic | added CD | OD (620 nm) | turbidity (%) | clearness (%) |
|---|---|---|---|---|
| 0.2% dibucaine (pH 7.25) | | 2.015 | 100 | 0 |
| | 0.51% α-CD | 0.017 | 0.84 | 99.16 |
| | 0.60% β-CD | 0.032 | 1.59 | 98.41 |
| | 0.63% γ-CD | 0.299 | 14.84 | 85.16 |
| 0.5% tetracaine (pH 7.75) | | 2.732 | 100 | 0 |
| | 1.62% α-CD | 0.817 | 29.91 | 70.09 |
| | 1.89% β-CD | 0.001 | 0.04 | 99.96 |
| 0.5% bupivacaine (pH 7.25) | | 2.108 | 100 | 0 |
| | 1.50% α-CD | 0.562 | 26.66 | 73.34 |
| 2.0% lidocaine (pH 7.75) | | 2.141 | 100 | 0 |
| | 7.18% α-CD | 0.813 | 37.97 | 62.03 |
| | 11.08% HP-β-CD | 0.016 | 0.75 | 99.25 |
| | 12.28% HP-γ-CD | 0.134 | 6.26 | 93.74 |
| OD (620 nm) means absorbance determined at wavelength of 620 nm. | | | | |

As shown in Table 1, the inclusion compound solution of a local anesthetic in cyclodextrin or a derivative thereof showed lower turbidity and higher clearness as compared to the solution of each local anesthetic. Thus, it has been shown that inclusion of a local anesthetic in cyclodextrin or a derivative thereof markedly increased the solubility of the local anesthetic at a pH near that of a living body.

Experimental Example 2

The absorbance at wavelength 620 nm [OD(620 nm)] and pH of 0.4% dibucaine solution were determined using UV-visible recording spectrophotometer (UV-2100, Shimadzu Corporation). The solution was stirred, and OD (620 nm) and pH were determined at 5 min, 24 hr, 48 hr and 72 hr later. The results are shown in Table 2.

In the same manner as in Example 1, to 0.4% dibucaine solution was added $\alpha$-cyclodextrin having an equimolar concentration to give an inclusion compound solution. OD (620 nm) and pH of the inclusion compound solution were determined in the same manner as above. The solution was stirred, and OD (620 nm) and pH were determined. The results are shown in Table 2.

In the same manner as above, OD (620 nm) and pH of 1.0% tetracaine solution and an inclusion compound solution prepared by adding hydroxypropyl-$\beta$-cyclodextrin having an equimolar concentration to 1.0% tetracaine solution were determined. The solutions were stirred, and OD (620 nm) and pH were determined. The results are shown in Table 2.

In the same manner as above, OD (620 nm) and pH of 0.5% bupivacaine solution and an inclusion compound solution prepared by adding $\alpha$-cyclodextrin having an equimolar concentration to 0.5% bupivacaine solution were determined. The solutions were stirred, and OD (620 nm) and pH were determined. The results are shown in Table 2.

In the same manner as above, OD (620 nm) and pH of 5.0% lidocaine solution and an inclusion compound solution prepared by adding hydroxypropyl-$\beta$-cyclodextrin having an equimolar concentration to 5.0% lidocaine solution were determined. The solutions were stirred, and OD (620 nm) and pH were determined. The results are shown in Table 2.

Table 2

| local anesthetic | time | | | | |
|---|---|---|---|---|---|
| | initial | 5 min later | 24 hr later | 48 hr later | 72 hr later |
| 0.4% dibucaine | 0.000 | 1.528±0.087 | 1.456±0.079 | 1.385±0.042 | 1.412±0.054 |
| | 6.19 | 6.85 | 6.76 | 6.73 | 6.75 |
| 0.4% dibucaine+ 1.02% $\alpha$-CD | 0.000 | 0.000 | 0.000 | 0.000 | 0.000 |
| | 6.14 | 7.20 | 7.20 | 7.17 | 7.12 |
| 1.0% tetracaine | 0.000 | 0.517±0.024 | 0.411±0.024 | 0.386±0.015 | 0.301±0.025 |
| | 5.49 | 7.12 | 7.04 | 7.05 | 7.05 |
| 1.0% tetracaine+ 5.00% HP-$\beta$-CD | 0.009 | 0.010±0.000 | 0.011±0.001 | 0.011±0.001 | 0.011±0.001 |
| | 5.44 | 7.20 | 7.20 | 7.22 | 7.22 |
| 0.5% bupivacaine | 0.000 | 0.087±0.015 | 0.659±0.084 | 0.247±0.061 | |
| | 5.44 | 7.20 | 6.89 | 6.77 | |
| 0.5% bupivacaine+ 1.50% $\alpha$-CD | 0.000 | 0.001±0.000 | 0.002±0.001 | 0.022±0.008 | |
| | 5.29 | 7.20 | 7.20 | 7.16 | |
| 5.0% lidocaine | 0.006 | 1.045±0.242 | 0.717±0.152 | 0.382±0.044 | 0.595±0.086 |
| | 4.59 | 7.09 | 7.05 | 7.10 | 7.12 |
| 5.0% lidocaine+ 27.7% HP-$\beta$-CD | 0.021 | 0.018±0.002 | 0.018±0.002 | 0.018±0.001 | 0.019±0.001 |
| | 5.99 | 7.20 | 7.20 | 7.22 | 7.20 |
| The upper value is absorbance at 620 nm (mean±S.E.) and lower value is pH (average). (N=4) | | | | | |

As is evident from Table 2, the solutions of local anesthetic became cloudy due to precipitation occurred immediately after stirring and showed increased absorbances. In contrast, the solutions of local anesthetic included in cyclodextrin or a derivative thereof maintained clearness of the solution even 48 hr to 72 hr later. Therefrom it has been shown that inclusion of a local anesthetic in cyclodextrin or a derivative thereof markedly increases its solubility which can be retained at a high level for a long time.

Experimental Example 3

Aqueous local anesthetic solutions having the following compositions were prepared.

| Formulation A (total amount 100 ml) | |
|---|---|
| lidocaine hydrochloride | 2 g |
| sodium salicylate | 1 g |
| 1N aqueous sodium hydroxide solution | q.s. |
| distilled water | q.s. |

| Formulation B (total amount 100 ml) | |
|---|---|
| hydroxypropyl-$\beta$-cyclodextrin | 10 g |
| lidocaine hydrochloride | 2 g |
| sodium salicylate | 1 g |
| 1N aqueous sodium hydroxide solution | q.s. |
| distilled water | q.s. |

| Formulation C (total amount 100 ml) | |
|---|---|
| dibucaine hydrochloride | 100 mg |
| sodium salicylate | 200 mg |
| 1N aqueous sodium hydroxide solution | q.s. |
| distilled water | q.s. |

| Formulation D (total amount 100 ml) | |
|---|---|
| dibucaine hydrochloride | 100 mg |
| sodium salicylate | 200 mg |
| $\alpha$-cyclodextrin | 300 mg |
| 1N aqueous sodium hydroxide solution | q.s. |
| distilled water | q.s. |

The above-mentioned aqueous local anesthetic solutions were examined for clearness of the solution with different pHs. The results are shown in Tables 3 and 4.

Table 3

|  | pH 6.0 | pH 7.5 |
|---|---|---|
| Formulation A | clear | cloudy white |
| Formulation B | clear | clear |

Table 4

|  | pH 5.0 | pH 7.5 |
|---|---|---|
| Formulation C | clear | cloudy white |
| Formulation D | clear | clear |

From these results, it has been found that a combination of inclusion of local anesthetic in cyclodextrin or a derivative thereof and addition of salicylic acid or a salt thereof results in markedly improved solubility in the same weak alkaline range as in a living body.

Experimental Example 4

Abdominal giant nerve bundle of crayfish (*Procambarus clarkii*) was removed and this nerve was immersed for 2 hr in various concentrations of single local anesthetic solutions (free of cyclodextrin or its derivative), which were specifically 0.03% dibucaine solution and 0.5% tetracaine solution dissolved in Harreveld solution (pH 6.5). Electrical stimulation was given from one end of each nerve removed and action potential was recorded at the other end to confirm that the action potential completely disappeared due to the anesthetic action. Then, anesthetic was removed from the nerve by immersing the nerve in normal Harreveld solution (physiological Ringer solution of crayfish) without a local anesthetic, while monitoring the recovery of action potential for 8 hr. The neurofunction could not be recovered with respect to any of the local anesthetic solutions used.

Recovery of neurofunction with respect to each local anesthetic used is shown in Table 5.

Table 5

| local anesthetic | | anesthetic effect | recovery |
|---|---|---|---|
| concentration | kind | disappearance of action potential after immersing nerve for 2 hr in anesthetic | observing appearance of action potential for maximum 8 hr after washing nerve |
| 0.03% | dibucaine | ○ | X |
| 0.5% | tetracaine | ○ | X |
| ○ : disappearance<br>X : no recovery | | | |

As shown in Table 5, neither dibucaine solution nor tetracaine solution recovered neurofunction even though the concentrations were low. In particular, dibucaine having potent local anesthetic effect was used at a concentration of one-tenth that actually used in clinical situations. However, dibucaine did not show recovery of action potential, indicating that it has strong neurotoxicity.

Experimental Example 5

α-Cyclodextrin, a derivative thereof (hydroxypropyl α-cyclodextrin) or β-cyclodextrin was added to a dibucaine solution (pH 6.5), and inclusion compound solutions were prepared in the same manner as in Example 1.

The obtained inclusion compound solutions of dibucaine in α-cyclodextrin, β-cyclodextrin or hydroxypropyl α-cyclodextrin were examined for expression of anesthetic effect and recovery in the same manner as in Experimental Example 4. The results which indicate neurotoxicity are shown in Table 6.

Table 6

| local anesthetic | | additive | | anesthetic effect | recovery |
|---|---|---|---|---|---|
| concentration | kind | concentration | kind | disappearance of action potential after immersing nerve for 2 hr in anesthetic | observing appearance of action potential for maximum 8 hr after washing nerve |
| 0.03% | dibucaine | 0.08% | α-CD | ○ | ◎ recovery in 30 min |
| | | 0.1% | hydroxypropyl α-CD | ○ | ◎ recovery in 30 min |
| | | 0.09% | β-CD | ○ | ◎ recovery in 6 hr |
| 0.1% | dibucaine | 0.25% | α-CD | ○ | ◎ recovery in 2 hr |
| | | 0.3% | hydroxypropyl α-CD | ○ | ◎ recovery in 3 hr |
| ○ : disappearance ◎ : recovery X : no recovery | | | | | |

It has been found that the inclusion compounds produced by the addition of the above-mentioned additives to dibucaine (pH 6.5) did not lose anesthetic action but retained the same. Then, dibucaine was washed away to examine recovery of neurofunction. As shown in Table 6, the neurofunction was found to have recovered. When the above-mentioned inclusion compound solutions of dibucaine were used, the function was recovered and the time necessary for the recovery became longer with higher dibucaine concentrations. This suggests possible control of sustained time of local anesthetic effect by increase/decrease of concentration and dose of local anesthetic, which is a noteworthy effect of α-cyclodextrin.

Experimental Example 6

β-Cyclodextrin was added to a tetracaine solution (pH 6.5) and an inclusion compound solution was prepared in the same manner as in Example 1.

Using the obtained inclusion compound solution of tetracaine in β-cyclodextrin, expression of anesthetic effect and recovery were examined in the same manner as in Experimental Example 4. The results which indicate neurotoxicity are shown in Table 7.

Table 7

| local anesthetic | | additive | | anesthetic effect | recovery |
|---|---|---|---|---|---|
| concentration | kind | concentration | kind | disappearance of action potential after immersing nerve for 2 hr in anesthetic | observing appearance of action potential for maximum 8 hr after washing nerve |
| 0.5% | tetracaine | 1.89% | β-CD | ○ | ◎ recovery in 3 hr |
| ○ : disappearance ◎ : recovery | | | | | |

It has been found that the inclusion compound produced by the addition of β-cyclodextrin to a tetracaine solution (pH 6.5) did not lose anesthetic effect but retained the same. Then, tetracaine was washed away to examine recovery of neurofunction. As shown in Table 7, the neurofunction was found to have recovered.

The results suggest decrease of neurotoxicity of tetracaine by the inclusion of tetracaine in β-cyclodextrin.

Experimental Example 7

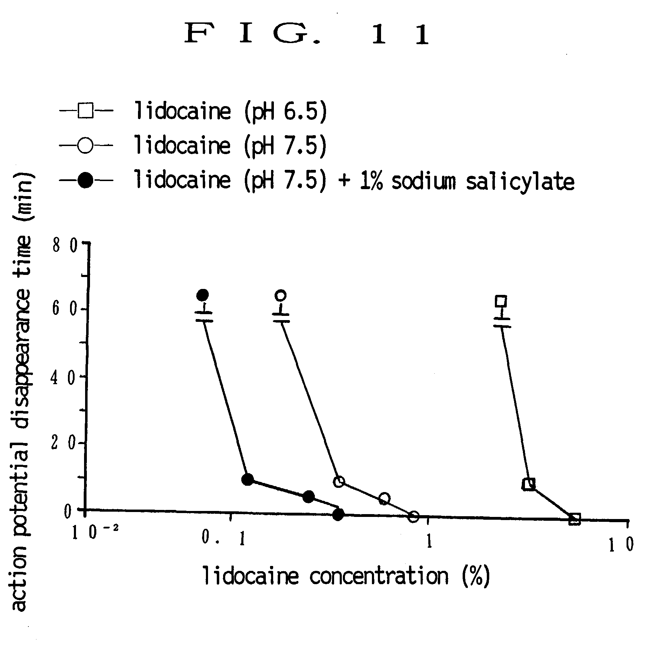

A local anesthetic effect is known to be stronger in alkaline range where pH value is greater. An abdominal giant nerve bundle of crayfish was removed and this nerve was immersed in a lidocaine solution (pH 6.5). The lidocaine concentration was changed from 2% to 5%, and the relationship between lidocaine concentration and action potential disappearance time was examined. The nerve extracted in the same manner as above was immersed in a lidocaine solution (pH 7.5). The lidocaine concentration was changed from 0.2% to 1%, and the relationship between lidocaine concentration and action potential disappearance time was examined. The results are shown in Fig. 11. In generality, lower lidocaine concentrations are associated with longer time necessary for action potential of the nerve to disappear due to anesthetic action, and higher concentrations are associated with shorter time. As shown in Fig. 11, however, 2% lidocaine (pH 6.5) required 80 min for the expression of anesthetic action, whereas at pH 7.5, action potential disappeared and anesthetic action was expressed in 2 min even at a concentration of 0.4%, which was lower than the former, thus indicating greater anesthetic effect in an alkaline range.

A nerve extracted in the same manner as above was immersed in a lidocaine solution (pH 7.5) containing 1% sodium salicylate. The lidocaine concentration was changed from 0.05% to 0.3%, and the relationship between lidocaine concentration and action potential disappearance time was examined. The results are shown in Fig. 11. The results show that the addition of salicylate led to a shortened time necessary for the expression of anesthetic action, as well as presence of anesthetic action at a lower anesthetic concentration.

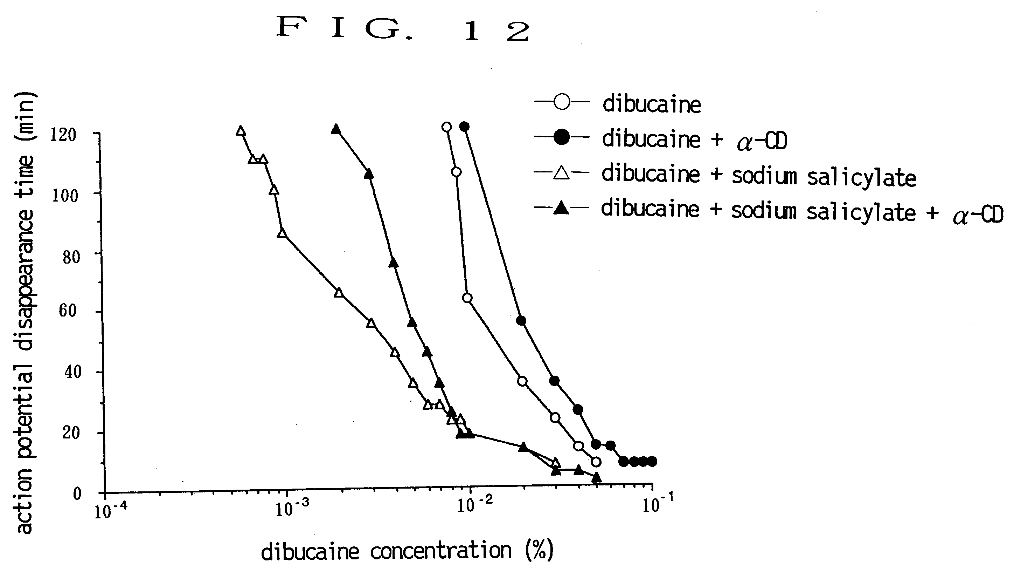

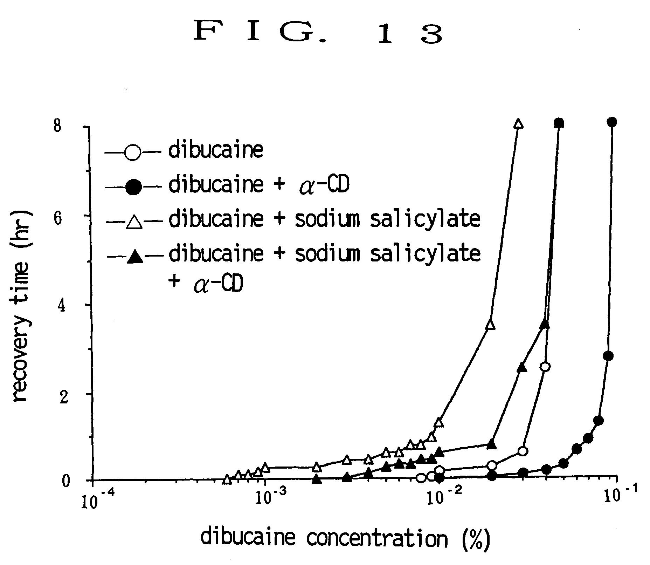

Then, anesthetic action was examined when local anesthetic enhancing effect of salicylate and anesthesia recovery improving effect of α-cyclodextrin were combined. α-CD and sodium salicylate were added to dibucaine, and in the same manner as in Experimental Example 4, the relationship between dibucaine concentration and neuroaction potential disappearance time was examined at pH 7.4. The results are shown in Fig. 12. In addition, the relationship between dibucaine concentration and neuroaction potential recovery time was examined at pH 7.4. The results are shown in Fig. 13. When dibucaine alone was administered at pH 7.4, action potential disappeared and recovered at a dibucaine concentration of 0.008%-0.05%, whereas the addition of α-CD having an equimolar concentration transferred the dibucaine concentration necessary for disappearance and recovery of action potential to a higher concentration side of 0.01%-0.1%. Further addition of 0.05% sodium salicylate transferred the dibucaine concentration to a lower concentration of 0.002%-0.05%. When 0.05% sodium salicylate alone was added to dibucaine, disappearance and recovery of action potential occurred at an extremely low dibucaine concentration of 0.0006%-0.03%.

It has been found that, while salicylic acid promotes anesthetic action and delays recovery, α-cyclodextrin conversely delays anesthetic action and promotes recovery. When they are used in combination, they are in preferable mutual relationship in that salicylic acid compensates for delay in anesthesia expression due to α-cyclodextrin, and delay in anesthesia recovery time due to salicylic acid is compensated for by α-cyclodextrin. This is effective in improving neurotoxicity so far understood to damage neurofunction for good when a potent anesthetic is used.

Experimental Example 8

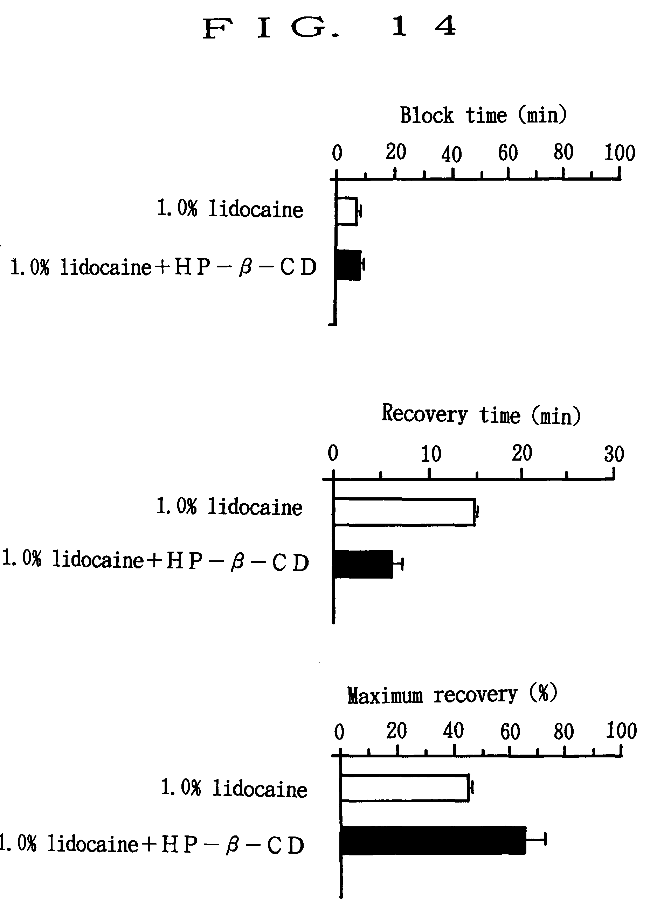

An abdominal giant nerve bundle of crayfish was extracted and immersed in a test solution prepared by adding lidocaine solution (1.0%) to Harreveld solution (pH 7.4), and action potential was recorded. The time when the amplitude of the action potential became 10% or less (mean±S.E.) of the amplitude at 0 min was taken as the action potential disappearance time. After immersion of the nerve bundle for 2 hr in the test solution, it was transferred into the Harreveld solution (pH 7.4), and the time necessary for the amplitude of the action potential to be not less than 10% (mean±S.E.) was taken as the recovery time. The maximum amplitude (mean±S.E.) of action potential during recovery period (2-8 hr) was determined, and expressed in a relative value to the amplitude of action potential at 0 min which was taken as 100. This relative value was taken as the maximum recovery (%) of neurofunction. The results are shown in Fig. 14.

Then, hydroxypropyl-β-cyclodextrin having an equimolar concentration was added to 1.0% lidocaine solution, and an inclusion compound solution was prepared in the same manner as in Example 1. The inclusion compound solution of lidocaine in hydroxypropyl-β-cyclodextrin was added to Harreveld solution (pH 7.4). The aodominal giant nerve bundle of crayfish was immersed in this solution, and action potential disappearance time (mean±S.E.), recovery time (mean± S.E.) and maximum recovery (mean±S.E.) were determined in the same manner as above. The results are shown in Fig. 14.

When compared to a case where lidocaine was used alone, inclusion of lidocaine in hydroxypropyl-β-cyclodextrin led to a shortened recovery time and an increased maximum recovery. These results indicate that inclusion of lidocaine in cyclodextrin or a derivative thereof results in less neurotoxicity and significant retention of neurofunction.

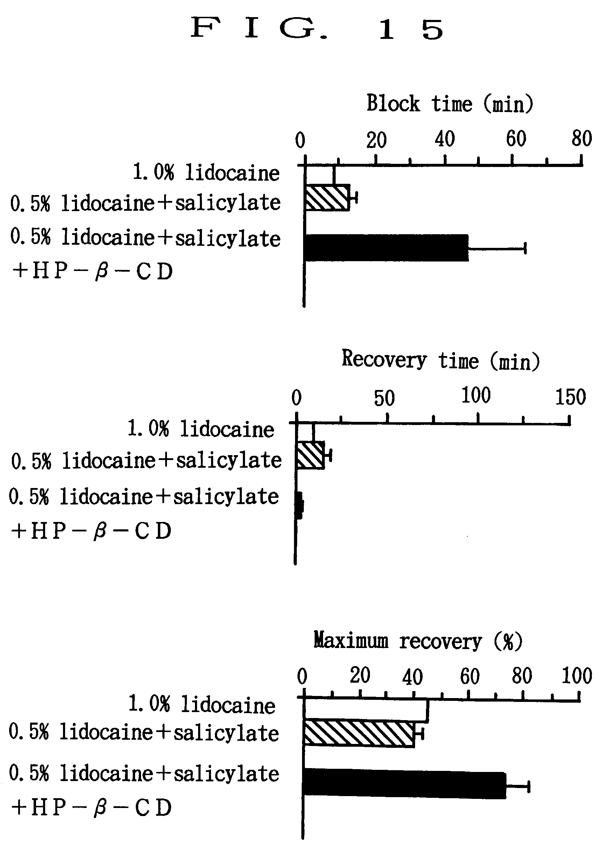

Then, sodium salicylate was added to a test solution containing lidocaine solution (0.5%) in Harreveld solution (pH 7.4), in a concentration of 0.05%. An abdominal giant nerve bundle of crayfish was immersed in this solution, and the action potential disappearance time (mean±S.E.), recovery time (mean±S.E.) and maximum recovery (mean± S.E.) were determined in the same manner as above. The results are shown in Fig. 15.

Then, hydroxypropyl-β-cyclodextrin having an equimolar concentration was added to a 0.5% lidocaine solution, and inclusion compound solutions were prepared in the same manner as in Example 1. The inclusion compound solution of lidocaine in hydroxypropyl-β-cyclodextrin was added to Harreveld solution (pH 7.4) and sodium salicylate was added thereto in a concentration of 0.05%. An abdominal giant nerve bundle of crayfish was immersed in this solution, and action potential disappearance time (mean±S.E.), recovery time (mean± S.E.) and maximum recovery (mean±S.E.) were determined in the same manner as above. The results are shown in Fig. 15.

When sodium salicylate was added to an inclusion compound of lidocaine in hydroxypropyl-β-cyclodextrin, greater extension of action potential disappearance time, slight shortening of recovery time and noticeable increase in maximum recovery were found, as compared to the addition of sodium salicylate to a lidocaine single solution. These results indicate that the action potential disappearance time was extended as a result of the lidocaine concentration which was reduced to half, but the addition of sodium salicylate and inclusion in cyclodextrin obliterated change in recovery time and afforded fine retention of neurofunction.

Experimental Example 9

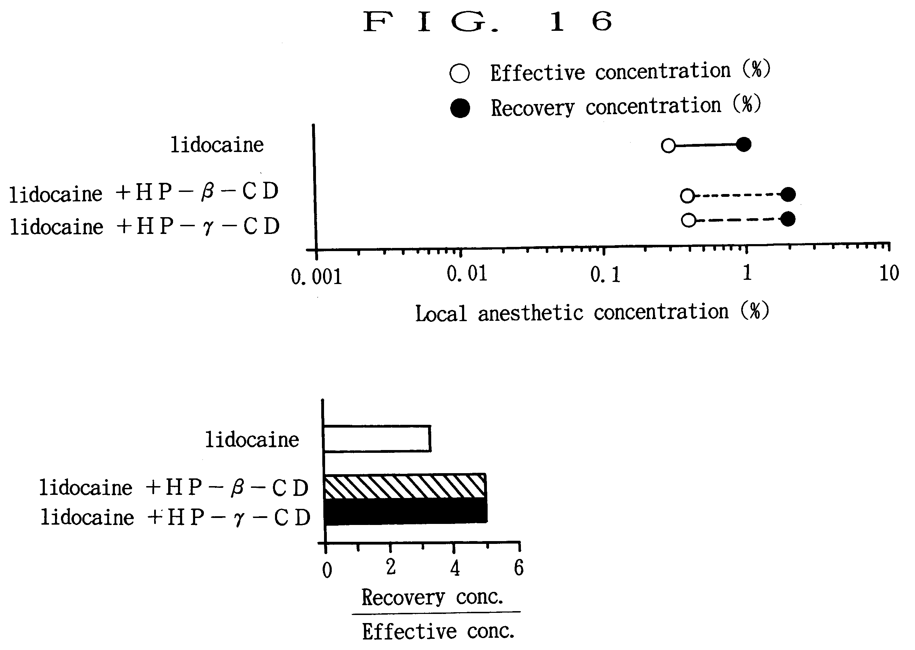

An abdominal giant nerve bundle of crayfish was extracted and immersed in a test solution prepared by adding a lidocaine solution to Harreveld solution (pH 7.4), and action potential was recorded. Lidocaine concentration was sequentially decreased and the concentration, at which action potential completely disappeared or reached an amplitude of not more than 10% of the initial amplitude within 2 hr, was taken as the minimum anesthesia effective concentration. Also, lidocaine concentration was sequentially increased, and the concentration, at which the action potential amplitude became not less than 10% within 8 hr, indicating recovery of impulse conduction, was taken as a maximum recovery concentration. Then, a safety ratio, which is a ratio of maximum recovery concentration to minimum anesthesia effective concentration, was calculated (safety ratio=maximum recovery concentration/minimum anesthesia effective concentration). This safety ratio is considered to be an index of the safety of local anesthetic when it is used in clinical situations. The results are shown in Fig. 16.

An inclusion compound solution of lidocaine in hydroxypropyl-β-cyclodextrin, obtained in the same manner as in Experimental Example 8, was added to Harreveld solution (pH 7.4), and abdominal giant nerve bundle of crayfish was immersed in this solution. Anesthesia effective concentration, recovery concentration and safety ratio were determined in the same manner as above. The results are shown in Fig. 16.

Hydroxypropyl-γ-cyclodextrin having an equimolar concentration was added to a lidocaine solution, and an inclusion compound solution was prepared in the same manner as in Example 1. The inclusion compound solution of lidocaine in hydroxypropyl-γ-cyclodextrin was added to Harreveld solution (pH 7.4). An abdominal giant nerve bundle of crayfish was immersed in this solution, and minimum anesthesia effective concentration and maximum recovery concentration were determined, and safety ratio was calculated in the same manner as above. The results are shown in Fig. 16.

When an inclusion compound of lidocaine in hydroxypropyl-β-cyclodextrin or hydroxypropyl-γ-cyclodextrin was used after addition to a Harreveld solution, the safety ratio increased 1.5-fold as compared to a Harreveld solution containing lidocaine alone. This result indicates that the inclusion of lidocaine in cyclodextrin or a derivative thereof results in decreased neurotoxicity and a local anesthetic agent having high safety.

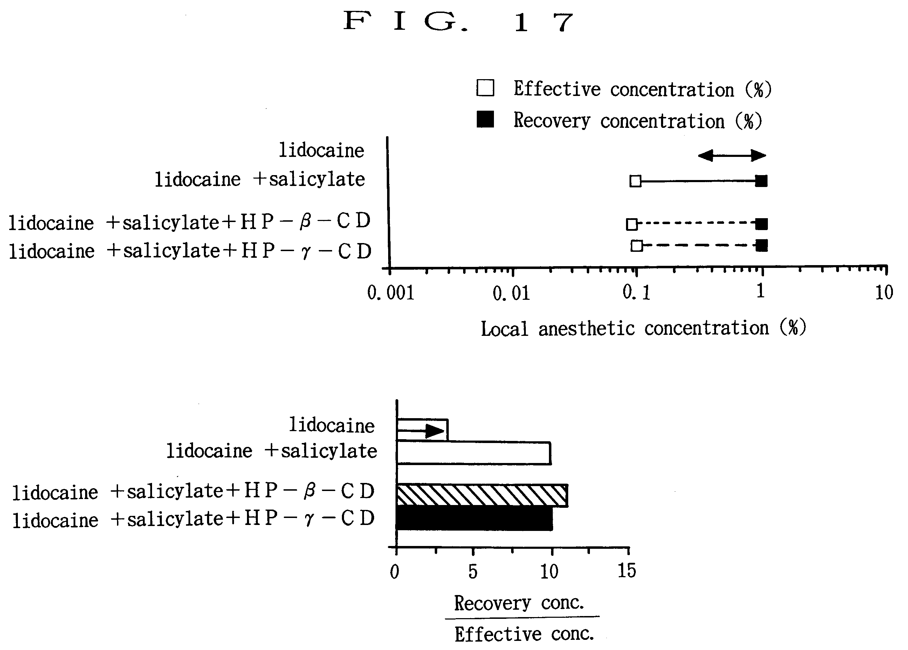

Then, sodium salicylate was added to a lidocaine solution in Harreveld solution (pH 7.4), in a concentration of 0.05%. An abdominal giant nerve bundle of crayfish was immersed in this solution, and minimum anesthesia effective concentration, maximum recovery concentration and safety ratio were obtained in the same manner as above. The results are shown in Fig. 17.

An inclusion compound solution of lidocaine in hydroxypropyl-β-cyclodextrin, which was obtained in the same manner as in Experimental Example 8, was added to Harreveld solution (pH 7.4) and sodium salicylate was added in a concentration of 0.05%. An abdominal giant nerve bundle of crayfish was immersed in this solution, and minimum anesthesia effective concentration, maximum recovery concentration and safety ratio were obtained in the same manner as above. The results are shown in Fig. 17.

An inclusion compound solution of lidocaine in hydroxypropyl-γ-cyclodextrin, which was obtained in the same manner as above, was added to Harreveld solution (pH 7.4) and sodium salicylate was added in a concentration of 0.05%. An abdominal giant nerve bundle of crayfish was immersed in this solution, and minimum anesthesia effective concentration, maximum recovery concentration and safety ratio were obtained in the same manner as above. The results are shown in Fig. 17.

When a Harreveld solution containing sodium salicylate and an inclusion compound of lidocaine in hydroxypropyl-β-cyclodextrin or hydroxypropyl-γ-cyclodextrin was used, minimum anesthesia effective concentration shifted towards lower concentration side, as compared to the results obtained with respect to a Harreveld solution containing lidocaine alone. The safety ratio increased about 3-fold as compared to the use of lidocaine alone. The results show that the inclusion of lidocaine in cyclodextrin or a derivative thereof combined with the addition of salicylic acid or a salt thereof strikingly widened the applicable range of lidocaine anesthesia and its effectiveness.

Experimental Example 10

While local anesthetics are known to show inhibitory action on the central nerve cells at high concentrations, they show stimulating action at low concentrations. Utilizing such action, the effect of the inventive anesthetic agent with less neurotoxicity was confirmed by the following acute toxicity test using mice. That is, 0.5 % dibucaine dissolved in physiological saline was subcutaneously injected into the back of 11 mice by 0.3 ml (40 mg/kg body weight), which were compared with 11 mice that underwent subcutaneous injection of a solution containing 0.5% dibucaine and $\alpha$-cyclodextrin having an equimolar concentration, by 0.3 ml (40 mg/kg body weight). The former mice showed jumping excitement soon after injection, tachycardia and rapid breathing, and then convulsion of extremities and respiratory distress, which ultimately resulted in cyanosis and death in about 20 min from the injection except one of them that escaped death. In contrast, the mice injected with dibucaine included in $\alpha$-cyclodextrin experienced similar symptoms, though of lighter levels, but did not develop cyanosis and recovered to normal in about 1.5 hr from the injection. However, 4 of them died. From the above acute toxicity test results, it has been found that the mortality which was 91% by administration of dibucaine alone was improved to 36% by the addition of $\alpha$-cyclodextrin, thus confirming effectiveness of $\alpha$-cyclodextrin in reducing neurotoxicity of local anesthetic.

Inasmuch as the local anesthetic contained in the aqueous local anesthetic solution of the present invention has an improved solubility by including same in cyclodextrin or a derivative thereof, it dissolves well at a pH similar to that of a living body. Thus, the inventive aqueous local anesthetic solution has a pH of 6.0-8.6, preferably 6.5-8.5, and can be used in a pH range similar to that of a living body. Therefore, the aqueous local anesthetic solution of the present invention is also effective in the tissue having an acidic pH, and reduces pain caused by conventional local anesthetic dissolved in acidic solution, at the injection site due to local acidosis.

According to the present invention, the local anesthetic agent containing an inclusion compound prepared by adding cyclodextrin or its derivative to a local anesthetic can temporarily stop the neurofunction by its anesthetic effect and certainly recover the function thereafter. This is clinically extremely beneficial for both patients and doctors. In addition, since neurotoxicity of a strong local anesthetic can be reduced, the concentration and dose of the anesthetic can be reduced/increased freely to control duration of the anesthesia. Moreover, since local anesthetic can be used in the same weak alkaline side with the living body, the local anesthetic agent of the present invention is expected to be particularly effective when living tissue has an acidic pH due to inflammation where anesthetic action of conventional anesthetics is weak. A sharp pain caused by local acidosis induced by a local anesthetic dissolved in acidic solution is expected to be reduced by dissolving the anesthetic in a weak alkaline solution.

According to the present invention, salicylic acid or a salt thereof is added to an inclusion compound obtained by adding cyclodextrin or a derivative thereof to a local anesthetic, whereby anesthetic effect is increased and a local anesthetic agent quickly expressing the anesthetic action for a prolonged time can be provided.

The local anesthetic agent of the present invention containing an inclusion compound of a local anesthetic in cyclodextrin or a derivative thereof has resistance to heat and can be used clinically, since it permits sterilization by heating. Thus, it can be formulated into injection, spray, jelly and the like which can be used widely in ophthalmology, dentistry and the like.

This application is based on application Nos. 284470/1996, 303284/1996 and 244093/1997 filed in Japan, the content of which is incorporated hereinto by reference.

## Claims

1. An aqueous local anesthetic solution having a pH of 6.5-8.5, which comprises a local anesthetic included in cyclodextrin or a derivative thereof in a concentration of the local anesthetic of not less than 0.02%.

2. The aqueous local anesthetic solution of claim 1, wherein the local anesthetic is lidocaine or a salt thereof which is contained in a concentration of the lidocaine or a salt thereof of not less than 0.1%.

3. The aqueous local anesthetic solution of claim 1, wherein the local anesthetic is bupivacaine or a salt thereof which is contained in a concentration of the bupivacaine or a salt thereof of not less than 0.1%.

4. The aqueous local anesthetic solution of claim 1, wherein the local anesthetic is dibucaine or a salt thereof which is contained in a concentration of the dibucaine or a salt thereof of not less than 0.02%.

5. The aqueous local anesthetic solution of claim 1, wherein the local anesthetic is tetracaine or a salt thereof which is contained in a concentration of the tetracaine or a salt thereof of not less than 0.02%.

6. The aqueous local anesthetic solution of any one of claims 1 to 5, further comprising salicylic acid or a salt thereof.

7. An external agent prepared using the aqueous local anesthetic solution of any one of claims 1 to 6.

8. The external agent of claim 7, which is an ointment, an adhesive plaster or a suppository.

9. A method for improving solubility of a local anesthetic in water at pH 6.5-8.5, comprising including the local anesthetic in cyclodextrin or a derivative thereof.

10. The method for improving solubility of claim 9, wherein the local anesthetic is at least one member selected from the group consisting of lidocaine, bupivacaine, dibucaine, tetracaine and salts thereof.

11. A Local anesthetic agent comprising an inclusion compound of a local anesthetic in $\alpha$-cyclodextrin or a derivative thereof.

12. The local anesthetic agent of claim 11 having a decreased neurotoxicity.

13. The local anesthetic agent of claim 11, wherein the local anesthetic is at least one member selected from the group consisting of dibucaine, tetracaine and salts thereof.

14. The local anesthetic agent of any one of claims 11 to 13, further comprising salicylic acid or a salt thereof.

15. A method of decreasing neurotoxicity of local anesthetic agent, comprising including the local anesthetic in $\alpha$-cyclodextrin or a derivative thereof.

16. An inclusion compound of local anesthetic in $\alpha$-cyclodextrin or a derivative thereof.

17. The inclusion compound of claim 16, wherein the local anesthetic is at least one member selected from the group consisting of dibucaine, tetracaine and salts thereof.

18. A local anesthetic agent comprising an inclusion compound of tetracaine or a salt thereof in $\beta$-cyclodextrin or a derivative thereof.

19. The local anesthetic agent of claim 18, having a decreased neurotoxicity.

20. The local anesthetic agent of claim 18 or 19, further comprising salicylic acid or a salt thereof.

21. A method of decreasing neurotoxicity of tetracaine or a salt thereof, comprising including tetracaine or a salt thereof in $\beta$-cyclodextrin or a derivative thereof.

22. An inclusion compound of tetracaine or a salt thereof in $\beta$-cyclodextrin or a derivative thereof.

23. A local anesthetic agent comprising an inclusion compound of lidocaine or a salt thereof in $\beta$-cyclodextrin, $\gamma$-cyclodextrin or a derivative thereof.

24. The local anesthetic agent of claim 23, having a decreased neurotoxicity.

25. The local anesthetic agent of claim 23 or 24, further comprising salicylic acid or a salt thereof.

26. A method of decreasing neurotoxicity of lidocaine or a salt thereof, comprising including lidocaine or a salt thereof in $\beta$-cyclodextrin, $\gamma$-cyclodextrin or a derivative thereof.

27. An inclusion compound of lidocaine or a salt thereof in $\beta$-cyclodextrin, $\gamma$-cyclodextrin or a derivative thereof.

# F I G. 1

## dibucaine （0. 2 5 mM) ＋α－C D

EP 0 838 225 A2

# F I G. 2

### dibucaine (0. 25mM) +α−CD

EP 0 838 225 A2

# F I G. 3

dibucaine (0. 2 5 mM) + β − C D

EP 0 838 225 A2

# F I G. 4

## dibucaine (0.25mM) + $\beta$-CD

# F I G. 5

dibucaine (0. 2 5 mM) + γ−C D

# F I G. 6

tetracaine（0.05mM）+α−CD

EP 0 838 225 A2

# F I G.  7

tetracaine （0. 0 5 mM）＋α－C D

EP 0 838 225 A2

# FIG. 8

tetracaine (0.05 mM) + $\beta$-CD

EP 0 838 225 A2

# F I G. 9

tetracaine （0．０５mM）＋ $\beta$ －CD

EP 0 838 225 A2

# FIG. 10

# F I G. 1 1

- —□— lidocaine (pH 6.5)
- —○— lidocaine (pH 7.5)
- —●— lidocaine (pH 7.5) + 1% sodium salicylate

F I G. 1 2

action potential disappearance time (min)

dibucaine concentration (%)

—○— dibucaine
—●— dibucaine + $\alpha$-CD
—△— dibucaine + sodium salicylate
—▲— dibucaine + sodium salicylate + $\alpha$-CD

EP 0 838 225 A2

# F I G. 1 3

# FIG. 14

# F I G.  1 5

Block time (min)

0    20    40    60    80

1. 0% lidocaine

0. 5% lidocaine+salicylate

0. 5% lidocaine+salicylate
+HP－β－CD

Recovery time (min)

0    50    100    150

1. 0% lidocaine

0. 5% lidocaine+salicylate

0. 5% lidocaine+salicylate
+HP－β－CD

Maximum recovery (%)

0    20    40    60    80    100

1. 0% lidocaine

0. 5% lidocaine+salicylate

0. 5% lidocaine+salicylate
+HP－β－CD

# F I G. 1 6

○ Effective concentration (%)

● Recovery concentration (%)

Local anesthetic concentration (%)

Recovery conc.
―――――――――
Effective conc.

EP 0 838 225 A2

# F I G. 1 7

☐ Effective concentration (%)
■ Recovery concentration (%)

lidocaine
lidocaine +salicylate
lidocaine +salicylate+HP−β−CD
lidocaine +salicylate+HP−γ−CD

0.001 0.01 0.1 1 10

Local anesthetic concentration (%)

lidocaine
lidocaine +salicylate
lidocaine +salicylate+HP−β−CD
lidocaine +salicylate+HP−γ−CD

0 5 10 15

$$\frac{\text{Recovery conc.}}{\text{Effective conc.}}$$

EP 0 838 225 A2